# EUROPEAN PATENT APPLICATION

(11) **EP 2 253 455 A1**
(43) Date of publication of application: **24.11.2010**
(21) Application number: 08869627.3
(22) Date of filing: 19.11.2008
(51) Int. Cl.: B29C 53/24, A61F 13/15, A61F 13/49, A61F 13/511, B29C 51/08

(54) **SHEET SHAPING METHOD, AND SHEET MANUFACTURED BY THE METHOD**

(30) Priority: 10.01.2008 JP 2008003415
(71) Applicant: Uni-charm Corporation, Ehime 799-0111 (JP)
(72) Inventor: AZUMA, Hideki, Kanonji-shi Kagawa 769-1602 (JP); AKAKI, Kenichi, Kanonji-shi Kagawa 769-1602 (JP)
(74) Representative: Fitchett, Stuart Paul
(86) International application number: PCT/JP2008/070979
(87) International publication number: WO 2009/087820

(57) **Abstract**

The present invention aims to provide a method for forming a thermoplastic polymer sheet with an elasticized region adapted to be extensible and contractible in its thickness direction without an apprehension that the thermoplastic polymer sheet might be broken, and the sheet manufactured by this method.

The present invention provides a method for forming a thermoplastic polymer sheet 10 at a distance from an outer peripheral edge 12 thereof with an elasticized region 11 adapted to be extensible and contractible in a thickness direction of the sheet using stationery mold 5 and movable mold 6 each having a plurality of forming blades 2, **characterized in that** a plurality of the forming blades are configured so as to have projected cross-sectional shapes on the sheet defined by similar curved lines and/or similar flexural lines arranged around a center of similitude and extending in the thickness direction of the sheet from the stationery mold and the movable mold so as to be engaged one with another but not to come in contact one with another, a step of introducing the sheet between the stationery mold and the movable mold and a step of bring the forming blades of the stationery mold and the movable mold into engagement with one another to stretch the sheet between distal ends of each pair of the adjacent forming blades. The sheet manufactured by this method is also provided.

## Description

### TECHNICAL FIELD

The present invention relates to a method for forming thermoplastic polymer sheets with elasticized regions adapted to be extensible and contractible in their thickness directions and to the sheets manufactured by this method.

### RELATED ART

JP2000-342625A discloses a liquid-pervious inner sheet for a bodily fluid-absorbent article wherein the sheet is formed at a desired part with an elasticized region adapted to be extensible and contractible vertically with respect to the plane defined by the sheet. This elasticized region is defined by a plurality of gathers in the form of regression lines extending from a peripheral zone toward a central zone. According to the disclosure of JP2000-342625A, undesirable fine gathers would not be formed in the elasticized region and therefore wearers would not experience any feeling of incompatibility. In addition, this sheet may be advantageously used for disposable diaper since a concave space adapted to receive and retain feces is formed by the elasticized region.
PATENT DOCUMENT 1: JP2000-342625A

### DISCLOSURE OF THE INVENTION

### PROBLEM TO BE SOLVED BY THE INVENTION

The elasticized region disclosed in JP2000-342625A may be obtained by subjecting the sheet to gear-processing. However, deep-draw work using gear-process may be accompanied with problems such as so-called seizing phenomenon and sheet breakage due to interference between gears. In view of such problems, it is a principal object of the present invention to provide a method for forming sheets with elasticized regions adapted to be extensible and contractible in thickness directions of the sheets without the foregoing problems, on one hand, and the sheet manufactured by this method, on the other hand.

### MEASURE TO SOLVE THE PROBLEM

The object set forth above is achieved, according to the present invention, by a method for forming a thermoplastic polymer sheet at a distance from an outer peripheral edge thereof with an elasticized region adapted to be extensible and contractible in a thickness direction of the sheet using stationery mold and movable mold each having a plurality of forming blades, wherein the method is **characterized in that** the plurality of forming blades are configured so as to have projected cross-sectional shapes on the sheet defined by similar curved lines and/or similar flexural lines arranged around a center of similitude and to extend in the thickness direction of the sheet from the stationery mold and the movable mold so as to be engaged one with another but not to come in contact one with another, and the method comprises a step of introducing the sheet between the stationery mold and the movable mold and a step of bring the forming blades of the stationery mold and the movable mold into engagement with one another to stretch the sheet between distal ends of each pair of the adjacent forming blades.

The term "curved lines and/or flexural lines" used herein should be understood to include closed curved lines and/or flexural lines (i.e., curved lines and/or flexural lines having neither origins nor end points) and partially devoid curved lines and/or flexural lines (curved lines and/or flexural lines having origins and end points).

For the forming method according to the present invention, the forming blades are preferably maintained in engaged state for a predetermined period. Alternatively, the forming blades are preferably maintained in engaged state for a predetermined period at a temperature lower than a fusion point of the thermoplastic polymer sheet but higher than an ambient temperature (JIS K 6900, 15 to 35°C). After the step of introducing the sheet, the sheet is preferably clamped in its thickness direction between the stationery mold and the movable mold in an outer peripheral region extending around a region in which the distal ends of the forming blades are arranged and/or a central region surrounded by the region in which the distal ends of the forming blades. Furthermore, the sheet can be formed with a partial region in which a depth of engagement is continuously changed when a plurality of forming blades are engaged one with another.

As the thermoplastic polymer sheet to be formed by the method according to the present invention, at least one of thermoplastic polymer film and thermoplastic polymer fibrous nonwoven fabric is used. Although "sheet" and "film" are sometimes distinguished from each other on the basis of thickness, film and sheet will not be distinguished on the basis of thickness so far as the present invention concerns and the term "sheet" is inclusive of both film and nonwoven fabric. According to the present invention, both films and nonwoven fabrics having elasticity may be used. The thermoplastic polymer sheet may comprise a laminated sheet consisting of two or more types of films and nonwoven fabrics, respectively.

### EFFECT OF THE INVENTION

The method according to the present invention utilizes engagement of a plurality of forming blades one with another to form an elasticized region and therefore the inventive method is free from the interference between the gears. In addition, the engagement depth of the forming blades may be adjusted to stretch the sheet without braking the sheet.

The forming blades may be maintained in engaged state for a predetermined period under heating or not heating to facilitate the elasticized region to be formed. The sheet may be clamped in its thickness direction between an outer peripheral region extending around a region in which the distal ends of the forming blades are arranged and/or between a central region surrounded by the region in the distal ends of the forming blades are arranged to prevent the sheet from being dragged between each pair of the adjacent forming blades when the forming blades are engaged one with another.

There may be provided a partial region in which the engagement depth continuously changes to adjust a shape presented by the elasticized region when the elasticized region is stretched in the thickness direction of the sheet. When the projected cross-sectional shapes of a plurality of the forming blades on the sheet are the partially devoid closed curved lines as has previously described, the engagement depths at the origins and the end points of such shape may be adjusted to be relatively shallow to avoid sheet breakage occurring in the vicinity of these origins and end points.

### BRIEF DESCRIPTION OF THE DRAWINGS

[FIG. 1] Perspective view showing a forming device as it is opened.
[FIG. 2] Schematic diagram illustrating a step of introducing a sheet between a stationary mold and a movable mold.
[FIG. 3] Schematic diagram illustrating a step of clamping the sheet between a pair of outer peripheral frames.
[FIG. 4] Schematic diagram illustrating a step of forming the sheet with an elasticized region.
[FIG. 5] Schematic diagram illustrating a part of Fig. 4 in an enlarged scale.
[FIG. 6] Perspective view of the sheet formed with the elasticized region.
[FIG. 7] Schematic diagram illustrating a cross-sectional shape of the sheet formed with the elasticized region.
[FIG. 8] Diagram exemplarily illustrating projected cross-sectional shapes of forming blades.
[FIG. 9] Perspective view of the stationery mold in the device according to a second embodiment of the invention.
[FIG. 10] Sectional view taken along a line X-X in Fig. 9.
[FIG. 11] Schematic diagram illustrating a manner in which the forming blades used for the method according to the second of the invention are engaged one with another.
[FIG. 12] Perspective view of the sheet for the method using the device according to the second embodiment of the invention.
[FIG. 13] Schematic diagram illustrating a cross-sectional shape of the elasticized region formed in the sheet by the method using the device according to the second embodiment of the invention.
[FIG. 14] Perspective view of a manufacturing equipment provided with the forming device.
[FIG. 15] Schematic diagram illustrating operation of the manufacturing equipment.
[FIG. 16] Schematic diagram how to control the operation of the manufacturing equipment.
[FIG. 17] Schematic diagram how to control the operation of the manufacturing equipment.
[FIG. 18] Schematic diagram how to control the operation of the manufacturing equipment.

### IDENTIFICATION OF REFERENCE NUMERALS USED IN THE DRAWINGS

- 2: forming blade
- 3: distal end of forming blade
- 4: engagement depth
- 5: stationary mold
- 6: movable mold
- 8: movement indicating imaginary straight line (thickness direction of sheet)
- 10: thermoplastic polymer sheet
- 11: elasticized region
- 12: outer peripheral edge

### DESCRIPTION OF THE BEST MODE FOR WORKING OF THE INVENTION

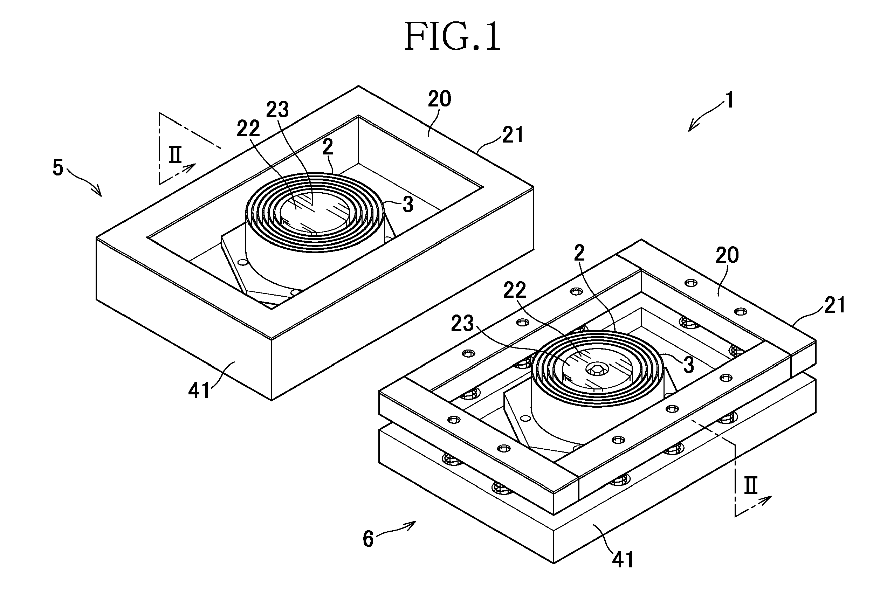

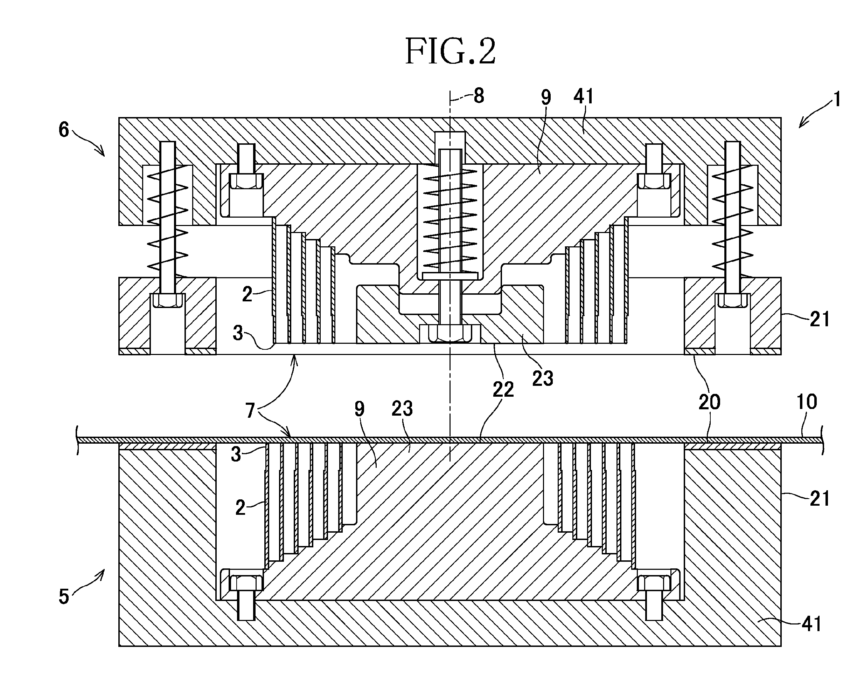

A method of forming a thermoplastic polymer sheet 10 (referred to hereafter simply as sheet 10) with an elasticized region 11 using a forming device 1 according to a first embodiment of the present invention provided with a plurality of forming blades 2 arranged in a pattern of concentric circles will be described hereinafter with reference to Figs. 1 through 5. Fig. 1 is a perspective view showing the forming device 1 in its opened state and Fig. 2 is a sectional view taken along a line II-II in Fig. 1, showing a stationery mold 5 and a movable mold 6 opposed to each other.

As shown by Fig. 1, the forming device 1 generally comprises the stationery mold 5 and the movable mold 6 both provided with a plurality of the forming blades 2 which are arranged in pattern of concentric circles, respectively. In other words, these forming blades 2 are arranged so that cross-sectional shapes thereof projected on the sheet 10 describe closed curves, respectively. In addition to these forming blades 2, the forming device 1 includes a pair of outer peripheral frames 21 and a pair of central frames 23. When the forming device 1 is operated, the movable mold 6 is opposed to the stationery mold 5 and moved by an actuator (not shown) along a movement indicating imaginary straight line 8 closer to or apart from the stationery mold 5, as will be appreciated from Fig. 2. In the course of forming, the movement indicating imaginary straight line 8 coincides with a thickness direction of the sheet 10.

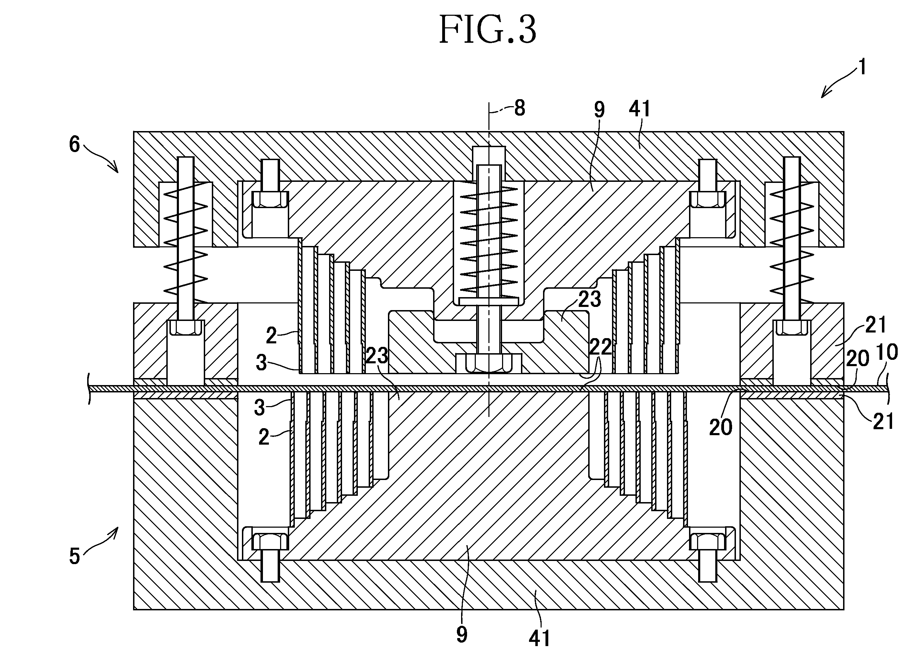

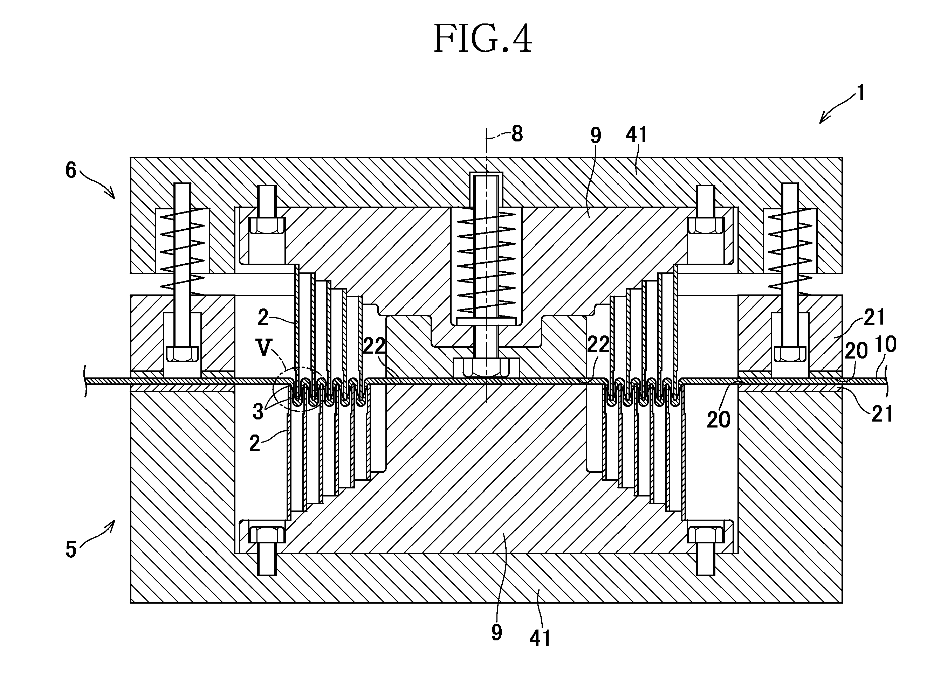

As will be apparent from Fig. 2, the forming blades 2 extend in parallel to the movement indicating imaginary straight line 8 and vertically mounted on respective pedestals 9, 9 provided within the stationery mold 5 and the movable mold 6. The forming blades 2 of the stationery mold 5 and the movable mold 6 are so arranged that these forming blades 2 may be engaged one with another but never brought in contact one with another as the movable mold 6 is moved closer to the stationery mold 6 along the movement indicating imaginary straight line 8 (See Figs. 3 and 4). Distal ends 3 of the forming blades 2 in both the stationery mold 5 and the movable mold 6 are coplanar, respectively, orthogonally to the movement indicating imaginary straight line 8. A distance between each pair of the adjacent forming blades 2 is larger than a thickness of the blade 2. Such dimensioning ensures that the forming blades 2 of the movable mold 6 are smoothly engaged with the forming blades 2 of the stationery mold 5 without contacting.

The opposed surfaces 7 of the movable mold 6 and the stationery mold 5 extending orthogonally to the movement indicating imaginary straight line 8 are defined by outer peripheral close-contact surfaces 20 of a pair of rectangular outer peripheral frames 21 disposed around a region in which the distal ends 3 of the forming blades 2 are arranged and a central close-contact surfaces 22 of a pair of circular central frames 23 disposed inside the region in which the distal ends 3 of the forming blades 2 are arranged. When the forming blades 2 of the movable mold 6 and the forming blades 2 of the stationery mold 5 are not engaged each other, the distal ends 3 of the forming blades 2 do not protrude outward beyond the outer peripheral close-contact surfaces 20 and the central close-contact surfaces 22. The outer peripheral frames 21 and the central frames 23 are mounted together with the respective pedestals 9 on foundations 41.

According the embodiment shown by Fig. 2, the outer peripheral frame 21 and the central frame 23 of the movable mold 6 are spring-biased to move toward the outer peripheral frame 21 and the central frame 23 of the stationery mold 5 until these outer peripheral close-contact surface 20 and central close-contact surface 22 come in close contact with those 20, 22 of the stationery mold 5, respectively. Alternatively, the outer peripheral frame 21 and the central frame 23 of the stationery mold 5 can be spring-biased to move toward those of the movable mold 6 or the outer peripheral frames 21 and the central frames 23 of both the stationery mold 5 and the movable mold 6 can be spring-biased to move toward those of the opposite molds 6, 5, respectively.

Shapes of the outer peripheral frame 21 and the central frame 23 are not limited to those exemplarily illustrated and may be appropriately selected depending on a contour of the region 11 to be elasticized. For example, the outer peripheral frame 21 may have an annular shape. It is also possible to eliminate the central frame 23. The outer peripheral close-contact surfaces 20 preferably protrude outward beyond the distal ends 3 of the forming blades 2 to prevent the sheet 10 from being unintentionally dragged into the gaps defined between each pair of the adjacent forming blades 2 engaged with each other and result in unacceptable forming, as will be described more in detail.

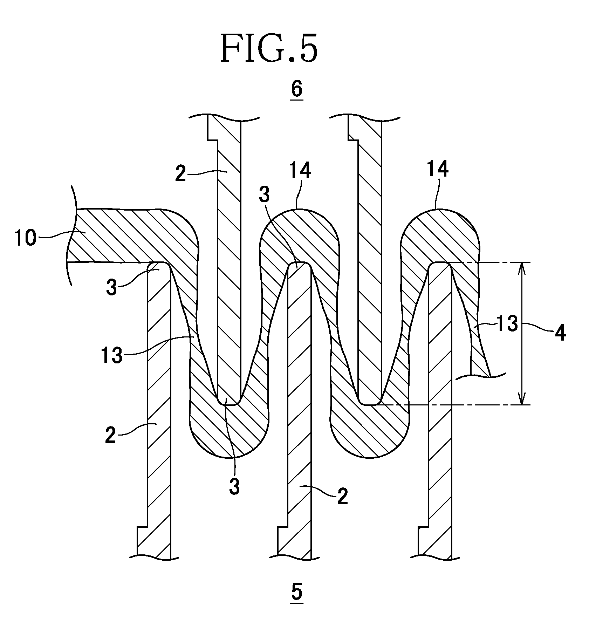

Now a process of forming the sheet 10 with the elasticized region 11 when the sheet 10 is in the form of film will be described with reference to Figs. 2 through 5. Fig. 2 illustrates a step of introducing the sheet 10 between the stationery mold 5 and the movable mold 6, Fig. 3 illustrates a step of firmly sandwiching the sheet 10 between the outer peripheral frames 21, 21 of the stationery mold 5 and the movable mold 6, and Fig. 4 illustrates a step of stretching the sheet 10 wherein the forming blades 2 come in engagement and thereby forming the sheet 10 with the elasticized region 11. Fig. 5 is a schematic diagram illustrating a part of Fig. 4 in an enlarged scale. Description given hereunder is on the assumption that the movable mold 6 moves along the movement indicating imaginary straight line 8 closer to and apart from the stationery mold 5.

In the step of introduction, the sheet 10 is introduced between the stationery mold 5 and the movable mold 6 as will be seen in Fig. 2. Upon movement of the movable mold 6 toward the stationery mold 5, the sheet 10 is firmly clamped between the outer peripheral close-contact surfaces 20, 20 of the outer peripheral frames 21, 21 of the stationery mold 5 and the movable mold 6 in the outer peripheral region extending around the region in which the distal ends 3 of the forming blades 2 are arranged, as illustrated by Fig. 3. In consequence, the sheet 10 is withheld from displacing in the direction orthogonal to the movement indicating imaginary straight line 8 and it is ensured that the sheet 10 is formed in a predetermined portion with the elasticized region 11.

Further movement of the movable mold 6 causes the forming blades 2 to come in engagement as illustrated by Fig. 4. When the forming blades 2 are engaged each other, the sheet 10 is clamped between the outer peripheral close-contact surfaces 20, 20 and between the central close-contact surfaces 22, 22 under a pressure and thereby withheld from displacing in the direction orthogonal to the movement indicating imaginary straight line 8. Consequently, the sheet 10 would not be unintentionally dragged into the gap defined between each pair of the adjacent forming blades 2 as the forming blades 2 come in engagement one with another. Specifically, the sheet 10 is stretched between each pair of the adjacent distal ends 3 of the forming blades 2 as schematically illustrated in an enlarged scale by Fig. 5, alternately creating high ratio-stretched zones 13 and low ratio-stretched zones 14. In this way, the elasticized region 11 is formed.

The forming blades 2 are preferably maintained in engaged state for a given period. The period for which the forming blades 2 should be maintained in engaged state may be appropriately set depending on an observed configuration of the elasticized region 11 which is being formed. The forming blades 2 may be maintained in engaged state for a given period at a temperature lower than a fusing point of the thermoplastic polymer but higher than an ambient temperature to facilitate the elasticized region 11 to be formed. Such temperature is usually in a range of temperature lower than the fusing point of the thermoplastic polymer by 20 to 30°C and may be appropriately selected taking account of on a thermal deformation temperature of the thermoplastic polymer such as a deflection temperature under load (JIS K 7191-2) or Vicat softening temperature (JIS K 7206) and depending on an observed configuration of the elasticized region 11 being formed.

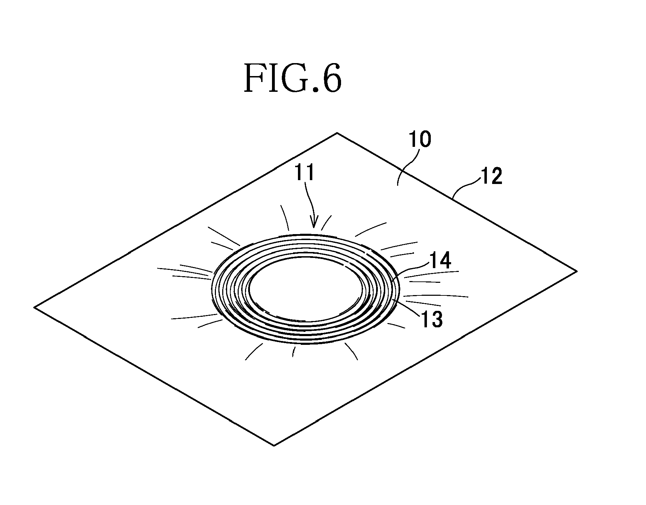

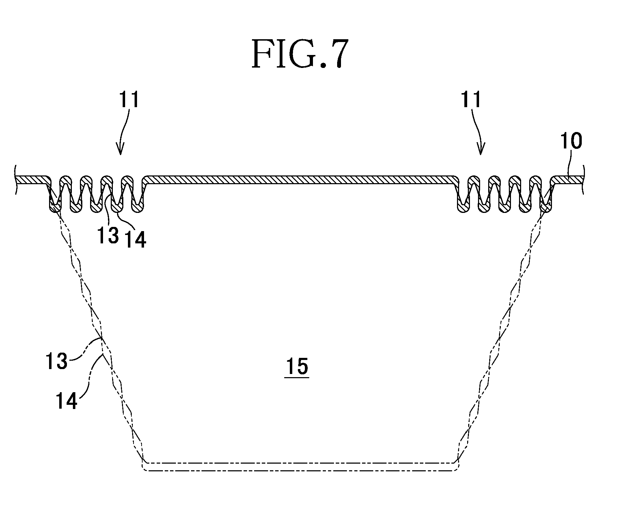

The sheet 10 formed with the elasticized region 11 in this manner is illustrated by Fig. 6 in perspective appearance and a cross-sectional shape thereof is schematically illustrated by Fig. 7. With this embodiment of the inventive device, the sheet 10 formed with the elasticized region 11 comprising a plurality of wavy undulations arranged in a concentric pattern is obtained as will be seen in Figs. 6 and 7. In the elasticized region 11, respective annular zones extending along crests of the respective wavy undulations are defined by the zones 14 stretched by the distal ends 3 of the forming blades 2 at the relatively low stretch ratio while respective belt-like zones extending along slopes of the respective wavy undulations are defined by the zones 13 stretched between each pair of the adjacent distal ends 3 of the forming blades 2 at the relatively high stretch ratio. As will be apparent from Fig. 7, the zones 14 stretched at the relatively low stretch ratio are correspondingly thick. In contrast with them, the high ratio-stretched zones 13 are thinner than the low ratio-stretched zones 14 and correspondingly more easily deformable than the zones 14.

In the elasticized region 11, the high ratio-stretched zones 13 and the low ratio-stretched zones 14 are alternately arranged. In response to a force exerted on the sheet 10 in the elasticized region 11 in its thickness direction, the respective high ratio-stretched zones 13 are immediately deformed in the direction of this force and the low ratio-stretched zones 14 follow the movement of the zones 13. Consequentially, the elasticized region 11 conically deploys in the thickness direction of the sheet 10 so as to form a space 15 which is concave with respect to the plane defined by the sheet 10, as shown in Fig. 7 with an imaginary line. When the sheet 10 having such elasticized region 11 is used for disposable diapers, this space 15 effectively serves to retain feces therein.

As will be understood from the foregoing description, with the forming method using the device 1 according to this embodiment, the sheet 10 would not be broken in the course of processing since the sheet 10 is formed with the elasticized region by stretching the sheet 10 in a range of stretch ratios acceptable for the thermoplastic polymer. In addition, the method using the device 1 according to this embodiment makes it possible to form the sheet 10 with the elasticized region 11 adapted to define the space 15 without need to attach a separate member to the sheet 10. Such sheet 10 may be used for disposable diapers adapted to retain feces to rationalize production of the diapers, for example, by decreasing the number of steps conventionally required to make the diaper.

While the method according to the invention has been described with respect to the case in which the sheet 10 is in the form of film, it should be appreciated that nonwoven fabrics made of thermoplastic polymer fiber may be also used as the sheet 10. When spun bond or melt blown nonwoven fabrics are used as the sheet 10, the high ratio-stretched zones 13 are more easily deformed than the low ratio-stretched zones 14 because heat bonds between fibers would be broken at the high ratio-stretched zones 13.

In addition to the thermoplastic polymer films and the thermoplastic fibrous nonwoven fabrics, it is possible to use initially elastic sheets such as films of thermoplastic elastomer or fibrous nonwoven fabrics of thermoplastic elastomer as the sheet 10. It is also possible to use various types of laminated sheets such as laminated sheets consisting of inelastic sheets and elastic sheets or laminated sheets comprising different types of inelastic sheets as the sheet 10. The thermoplastic polymer may be selected from a group including polyolefins, polyesters and polyamides and the thermoplastic elastomer may be selected from a group including those made of polyurethane- and polyester-based block copolymers.

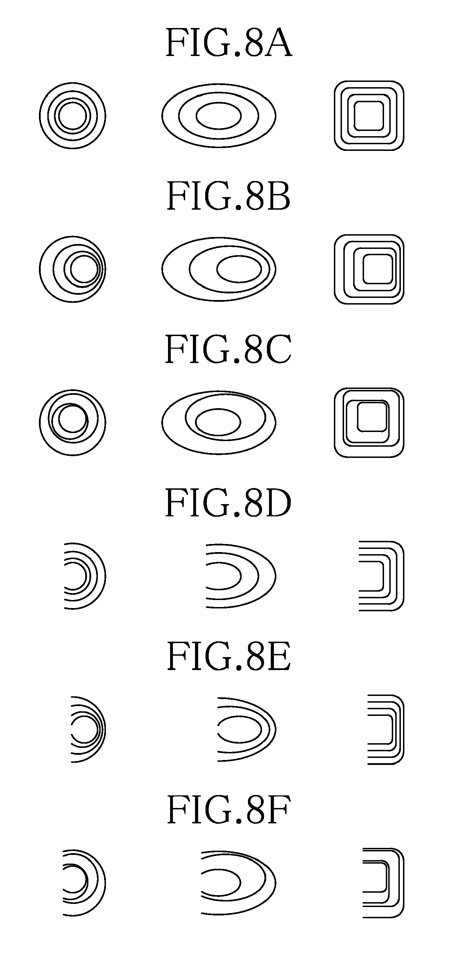

It should be understood that the high ratio-stretched zone 13 and the low ratio-stretched zone 14 in the elasticized region 11 formed according to the invention are not limited to those arranged in the pattern of concentric circles. Fig. 8 exemplarily illustrates cross-sectional shapes of the forming blades 2 projected on the plane extending orthogonally to the movement indicating imaginary straight line 8. These projected cross-sectional shapes correspond to possible patterns of the elasticized region 11 formed by the method according to the invention. More specifically, these possible patterns include a plurality of similar closed curved lines and/or flexural lines concentrically arranged in point symmetry about a center of similitude (Fig. 8A), a plurality of similar closed curved lines and/or flexural lines arranged concentrically and symmetrically about a straight line passing the center of similitude (Fig. 8B). Furthermore, the elasticized region 11 may present a pattern defined by a plurality of similar closed curved lines and/or flexural lines surrounding the center of similitude which is displaced aside insofar as these lines do not intersect one with another (Fig. 8C). Additionally, there may be various patterns defined by a plurality of similar closed but partially devoid curved lines surrounding the center of similitude (Fig. 8D - 8F).

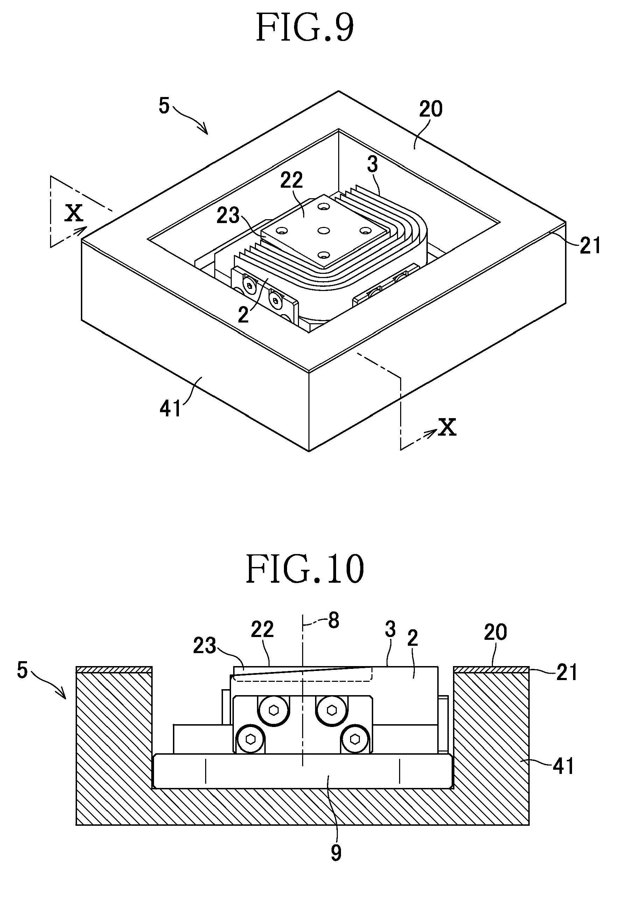

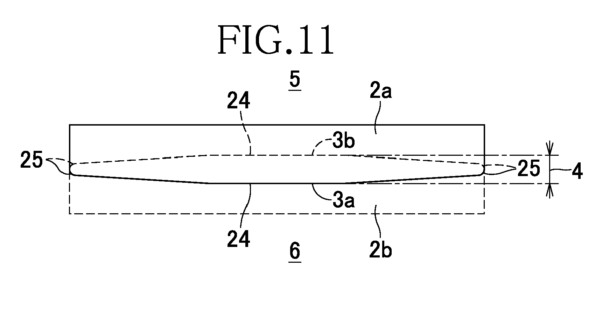

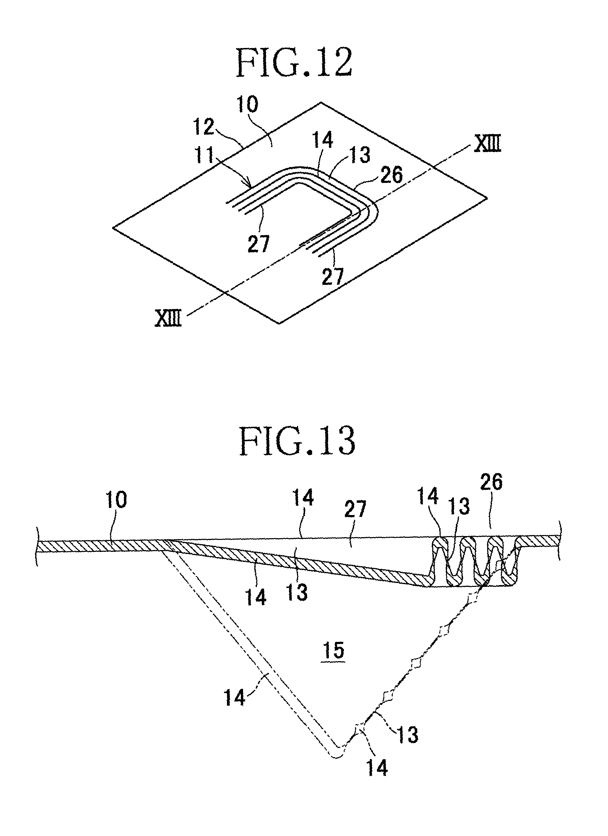

Now the method using the device 1 according to a second embodiment of the invention to form the elasticized region 11 comprising the high-ratio stretched zones 13 and the low-ratio stretched zones 14 alternately arranged so as to present a pattern defined by a plurality of similar closed but partially devoid curved lines surrounding the center of similitude will be described with reference to Figs. 9 through 18. Fig. 9 is a perspective view of the stationery mold 5 used in this embodiment, Fig. 10 is a sectional view taken along a line X-X in Fig. 9 and Fig. 11 is a schematic diagram illustrating a manner in which the forming blades 2 are engaged one with another when the movable mold 6 is engaged with the stationery mold 5. Fig. 12 is a perspective view of the sheet 10 formed with the elasticized region 11 and Fig. 13 is a diagram schematically illustrating a cross section of the elasticized region 11.

Description given hereinafter is on the assumption that the forming device 1 used for the forming method has the forming blades 2 arranged so that each partially devoid closed curved line describes a rectangle having its one side devoid (referred to hereinafter as semi-rectangle) and a plurality of similar such semi-rectangles surround the center of similitude and that the sheet 10 is used in the form of film.

Fig. 9 shows the stationery mold 5 as an important component of the forming device 1. Except that a plurality of forming blades 2 describe similar semi-rectangles, the stationery mold 5 used in this embodiment is similar to the stationery mold 5 used in the first embodiment and provided with the outer peripheral frame 21 and the central frame 23. The movable mold 6 used in this embodiment is similar to the stationery mold 5 except that the outer peripheral frame 21 and the central frame 23 are spring-biased to move toward the stationery mold 5 and is not illustrated. The features similar to those of the first embodiment will not be repetitively described in details.

As will be seen in Fig. 10, the distal ends 3 of the forming blades 2 do not protrude outward beyond the outer peripheral close-contact surface 20 of the outer peripheral frame 21 as well as beyond the central close-contact surface 22 of the central frame 23. It should be appreciated that the forming blades 2 of the stationery mold 5 extending in parallel to the movement indicating imaginary straight line 8 and are arranged so as to be engaged with the forming blades 2 of the movable mold 6 without contacting with the forming blades 2 of the movable mold 6.

Engagement of the forming blades 2 according to this embodiment to create the projected cross-sectional shape defined by a plurality of the partially devoid semi-rectangles is schematically illustrated by Fig. 11. In Fig. 11, reference numeral 2a represents a solid line connecting the forming blades 2 extending from the stationery mold 5 and reference numeral 2b represents a dashed line connecting the forming blades 2 extending from the movable mold 6. As will be seen in Fig. 11, the solid line connecting the distal ends 3a is parallel to the dashed line connecting the distal ends 3b in a middle segment 24 when the forming blades 2a, 2b are engaged with one another.

In lateral segments on both sides of the middle segment 24, a depth 4 at which the forming blades 2a, 2b are engaged one with another continuously decreases from the middle segment 24 toward the lateral segments 25. In consequence, the sheet 10 is prevented from being broken along corners of the forming blades 2a, 2b at the opposite end segments 25 in the course of forming the sheet 10 with the elasticized region 11 by the forming blades 2a, 2b. To prevent the sheet 10 from being broken, the forming blades 2a, 2b preferably have the round corners at the opposite end segments 25.

Between the movable mold 6 and the stationery mold 5 of the forming device 1 each having the forming blades 2 as have been described above, the sheet 10 is introduced and stretched between each pair of the adjacent distal ends 3 of the respective forming blades 2 to form the elasticized region 11 in a substantially same manner as in the first embodiment (See Figs. 2 through 5). As shown in Fig. 12, the elasticized region 11 formed by the method using the device 1 according to this embodiment presents an appearance defined by the semi-rectangles similar one to another alternately arranged around the center of similitude.

Fig. 13 is a schematic sectional diagram of the elasticized region 11 taken along a line XIII-XIII in Fig. 12. Of the elasticized region 11, the middle zone 26 stretched by the middle segments 24 of the forming blades 2 presents a corrugated cross-sectional shape comprising a plurality of high-ratio stretched zones 13 of uniform width arranged in parallel one to another. In the lateral zones 27 on both sides of the middle zone 26, the engagement depth 4 of the forming blades 2 gradually decreases and the width of the high-ratio stretched zones 13 formed between each pair of the adjacent forming blades 2 also gradually decreases as the forming blades 2 back away from the middle zone 26. As a result, the elasticized region 11 may be forced in the thickness direction of the sheet 10 to form a triangular space 15 which is concave from the plane defined by the sheet 10. In this manner, the projected cross-sectional shapes and the engagement depth 4 of the forming blades 2 may be appropriately changed to form the elasticized region 11 adapted to form the space 15 having desired shapes.

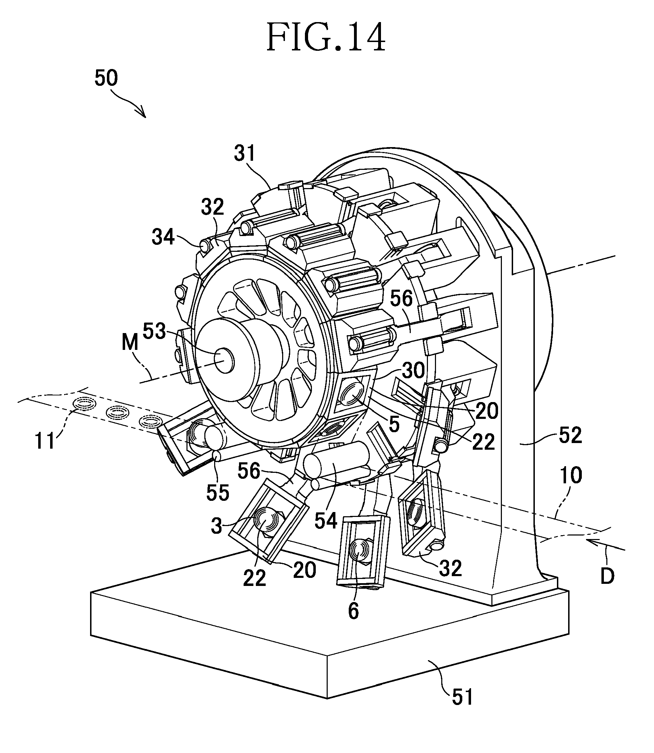

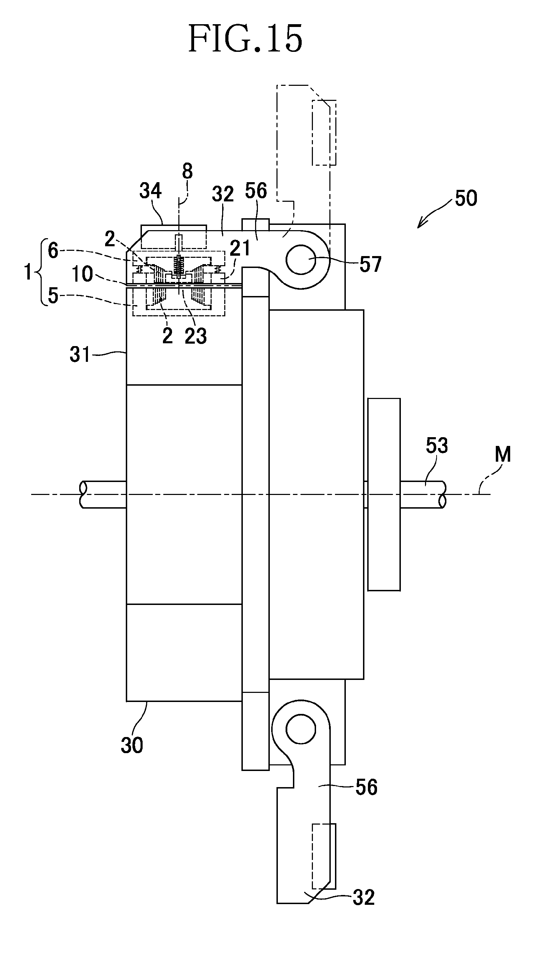

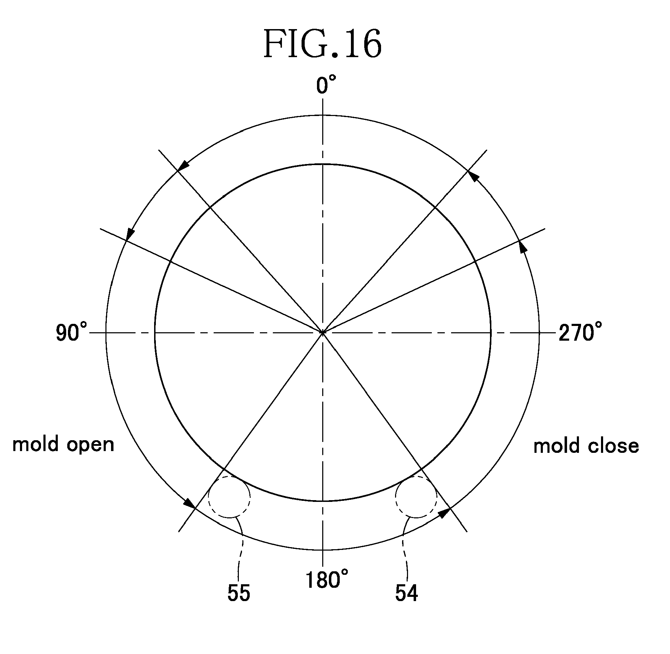

A manufacturing equipment 50 including the forming device 1 according to the first embodiment adapted to form the continuously fed sheet 10 with the elasticized region 11 and the method using this manufacturing equipment 50 will be described with reference to Figs. 14 through 18. Fig. 14 is a perspective view of the manufacturing equipment 50 provided with the forming device 1, Fig. 15 is a schematic side view illustrating operation of the forming device 1, and Figs. 16 through 18 are diagrams illustrating how to control the operation of the forming device 1 in the manufacturing equipment 50.

The manufacturing equipment 50 has a horizontal base 51 and a pair of columnar supports 52 vertically extending upward from the base 51 wherein a main shaft 53 horizontally extending between a pair of the columnar supports 52 on which a rotating drum 31 is rotatably mounted. The rotating drum 31 has a peripheral surface 30 extending in parallel to a rotational axis M indicated by an imaginary line. The sheet 10 indicated by an imaginary line is guided by the inlet nip roll pair 54 into contact with the peripheral surface 30 and guided by the outlet nip roll pair 55 to be moved apart from the peripheral surface 30. In Fig. 14, the equipment 50 is illustrated with one of the columnar supports 52 removed from the base 51.

A plurality of revolving heads 32 are provided at regular intervals around the rotating drum 31. The revolving heads 32 can be opposed to the peripheral surface 30 of the rotating drum 31. The revolving heads 32 revolve together with the rotating drum 31 around the rotational axis M in the direction indicated by an arrow D in which the sheet 10 is fed. The revolving heads 32 respectively have associated arms 56 are rotatably mounted on the rotating drum 31 (See Fig. 15) by the intermediary of associated spindles 57 (not shown in Fig. 14) extending orthogonally to the rotational axis M. Each of the revolving heads 32 is adapted to swing around the spindle 57 apart from its position opposed to the peripheral surface 30 to its completely opened position outside the rotating drum 31 under the effect of suitable revolving head driving means (not shown) mounted on the spindle 57. The completely opened position of the revolving head 32 is set so that the revolving head 32 is free from any interference with the sheet 10 when the revolving head 32 is revolving together with the rotating drum 31. As the revolving head driving means, the driving means of prior art such as a servomotor may be used.

A plurality of the stationery molds 5 as the important components of the forming device 1 are mounted on the peripheral surface 30 of the rotating drum 31 wherein various elements such as the distal ends 3 of the respective forming blades 2 and the outer peripheral close-contact surface 20 are exposed in each of the stationery molds 5. In association with the stationery molds 5, a plurality of the movable molds 6 are mounted on the respective revolving heads 32 so that each of these movable molds 6 may swing to its position opposed to the associated stationery mold 5. Both the movable mold 6 and the stationery mold 5 are similar to those having been described with respect to the first embodiment, i.e., the movement indicating imaginary straight line 8 coincides with the radial direction of the rotating drum 31 and the movable mold 6 may be brought into contact with the stationery mold 5 to engage the forming blades 2 of the movable mold 6 with the forming blades 2 of the stationery mold 5.

Each of the revolving heads 32 is provided with mold driving means 34 adapted to reciprocate the movable mold 6 along the movement indicating imaginary straight line 8 and thereby to engage the forming blades 2 of the movable mold 6 with the forming blades 2 of the associated stationery mold 5. As the mold driving means 34, a suitable actuator of prior art such as a servomotor or a hydraulic cylinder may be used. It is possible to dispose the movable mold 6 on the peripheral surface 30 of the rotating drum 31 and to dispose the stationery mold 5 on the revolving head 32 so that the stationery mold 5 may be reciprocated by the mold driving means 34.

The inlet nip roll pair 54 and the outlet nip roll pair 55 are respectively disposed on the upstream side and the downstream side as viewed in the direction in which the sheet 10 is fed. Both the inlet nip roll pair 54 and the outlet nip roll pair 55 extend in parallel to the rotational axis M and vertically underlie the rotational axis M. The sheet 10 is guided by the inlet nip roll pair 54 into contact with the stationery mold 5 mounted on the peripheral surface 30 of the rotating drum 31 and stretched between each pair of the adjacent distal ends 3 of the forming blades 2 as the forming blades 2 of the stationery mold 5 and the movable mold 6 come in engagement in the course of counterclockwise rotation of the drum 31 as viewed in drawings and thereby formed with the elasticized region 11. Then the sheet 10 is guided by the outlet nip roll pair 55 so as to move away from the peripheral surface 30, in other words, from the stationery mold 5.

The movable mold 6 of the forming device 1 mounted on the rotating drum 31 operates under a control as will be discussed on the basis of a position coordinate system as illustrated in Fig. 16 wherein an optional set of the stationery mold 5 and the movable mold 6 operatively associated with each other start from 0° position defined by the highest point of the rotating drum 31 to revolve counterclockwise by 360°. It should be appreciated that detection of the rotating drum's position and the engagement depth of the forming blades 2 as well as control of operation of the revolving head driving means 33 and the mold driving means 34 are carried out by a control unit (not shown).

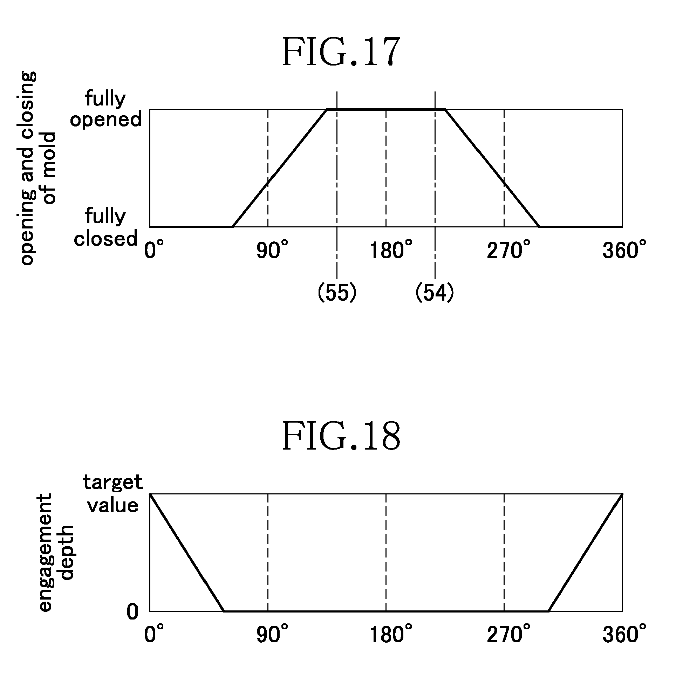

As will be apparent from Figs. 17 and 18, at the 0° position, the set of the molds 5, 6 are completely closed and an engagement depth of the forming blades 2 is set to a target value, i.e., the depth desired to stretch the sheet 10. In the course of revolving from 0° position toward 90° position, the mold driving means 34 nulls the engagement depth from the target value so as to release the movable mold 6 and subsequently the revolving head driving means 33 turns the movable mold 6 (the revolving head 32) to its completely opened position. The revolving head 32 should be completely opened before the revolving head 32 attains a position at which the outlet nip roll pair 55 is disposed (See Fig. 16) in order to prevent the revolving head 32 from interfering with the sheet 10.

The set of the molds 5, 6 revolve further beyond 180° position, passes the position of the inlet nip roll pair 54 (See Fig. 16) and the sheet 10 comes in contact with the peripheral surface 30 of the rotating drum 31. Thereupon operation to close the movable mold 6 is initiated and, after the set of the molds 5, 6 has passed 270° position, the movable mold 6 is completely closed with the sheet 10 clamped between the molds 5, 6. Then the mold driving means 34 is initiated so that the forming blades 2 of the movable mold 6 may be engaged with the forming blades 2 of the stationery mold 5 by the desired amount of engagement which is the target value (i.e., engagement depth 4) at 360° position, i.e., 0° position. As described above, the engagement depth of the forming blades 2 is preferably adjusted to attain the maximum value at 0° position to avoid undesirable phenomenon of seizing possibly occurring between each pair of the adjacent forming blades 2 of the stationery mold 5 and the movable mold 6.

The cycle as has been described above may be repeated to produce, in continuous fashion, the sheet formed with the elasticized regions 11 by using the manufacturing equipment 50. It should be noted here that the positions occupied by the set of the stationery mold 5 and the movable mold 6 in the position coordinate system have exemplarily described and not limited thereto.

## Claims

1. A method for forming a thermoplastic polymer sheet at a distance from an outer peripheral edge thereof with an elasticized region adapted to be extensible and contractible in a thickness direction of said sheet using stationery mold and movable mold each having a plurality of forming blades, said method is **characterized in that:**
said plurality of forming blades are configured so as to have projected cross-sectional shapes on said sheet defined by similar curved lines and/or similar flexural lines arranged around a center of similitude and extending in said thickness direction of said sheet from said stationery mold and said movable mold so as to be engaged one with another but not to come in contact one with another; and said method comprises:
a step of introducing said sheet between said stationery mold and said movable mold; and
a step of stretching said sheet wherein said forming blades of said stationery mold and said movable mold are brought into engagement with one another to stretch said sheet between distal ends of each pair of the adjacent forming blades.

2. The forming method according to Claim 1, wherein, in said step of stretching said sheet, said forming blades are maintained in engaged state for a predetermined period.

3. The forming method according to Claim 2, wherein, in said step of stretching said sheet, said forming blades are maintained in engaged state for a predetermined period at a temperature lower than a fusion point of said thermoplastic polymer sheet but higher than an ambient temperature.

4. The forming method according to any one of Claims 1 through 3, wherein, after said step of introducing said sheet, in an outer peripheral region extending around a region in which said distal ends of said forming blades are arranged and/or a central regions surrounded by said region in which said distal ends of said forming blades are arranged, said sheet is clamped in its thickness direction between said stationery mold and said movable mold.

5. The forming method according to any one of Claims 1 through 4, wherein there is provided a partial region in which a depth of engagement is continuously changed when said plurality of forming blades are engaged one with another.

6. Thermoplastic polymer sheet formed with said elasticized region by the forming method according to any one of Claims 1 through 5, wherein said thermoplastic polymer sheet comprises at least one of thermoplastic polymer films and thermoplastic polymer fibrous nonwoven fabrics.

7. Thermoplastic polymer sheet according to Claim 6, wherein at least one of said films and said nonwoven fabrics is elastic.

8. Thermoplastic polymer sheet according to Claim 6 or 7, wherein said thermoplastic polymer sheet comprises a laminated sheet consisting of two or more types of said films and said nonwoven fabrics, respectively.
